# EUROPÄISCHE PATENTSCHRIFT

(11) **EP 0 867 166 B1**
(45) Veröffentlichungstag und Bekanntmachung des Hinweises auf die Patenterteilung: **14.06.2006**
(21) Anmeldenummer: 98105606.2
(22) Anmeldetag: 27.03.1998
(51) Int. Cl.: A61K 6/083

(54) **Plaqueinhibierende Composite**
Dental plaque inhibiting composites
Matériaux composites inhibant la formation de plaque dentaire

(30) Priorität: 27.03.1997 DE 19713048
(43) Veröffentlichungstag der Anmeldung: 30.09.1998
(73) Patentinhaber: 3M ESPE AG, 82229 Seefeld (DE)
(72) Erfinder: Burger, Bernd, 82239 Alling (DE); Gangnus, Bernd, Dr., 82211 Herrsching (DE); Guggenberger, Rainer, Dr., 82211 Herrsching (DE)

(56) Entgegenhaltungen:
- WO-A-95/15740
- DE-A- 3 934 803
- DE-A- 4 445 266
- US-A- 4 738 722
- LOYOLA-RODRIGUEZ J P ET AL: "GROWTH INHIBITION OF GLASS IONOMER CEMENTS ON MUTANS STREPTOCOCCI" PEDIATRIC DENTISTRY, XX, US, Bd. 16, Nr. 5, 1. September 1994 (1994-09-01), Seiten 346-349, XP000576848 ISSN: 0164-1263

## Beschreibung

### Gebiet der Erfindung

Die Erfindung betrifft die Verminderung der Plaqueanlagerung an dentalen Werkstücken aus Kunststoffen.

Dentale Compositematerialien werden aufgrund ihrer guten ästhetischen und mechanischen Eigenschaften in der Zahnmedizin für ein breites Indikationsspektrum eingesetzt. Speziell bei der Verwendung im Bereich der Verblendung von Kronen, Brücken und Metallgerüsten für größere herausnehmbare und implantatgetragene Prothesen ist es von entscheidender ästhetischer und hygienischer Bedeutung, die im oralen Milieu auftretende Plaqueanlagerung so gering wie möglich zu halten. Dentale Compositematerialien neigen im Vergleich zu Dentalmaterialien auf Basis von Polymethylmethacrylat (PMMA) oder Keramik, wie beispielsweise Konfektionszähnen, zu einer erhöhten Plaqueanlagerung. Dahingegen wird für Dentalamalgam und Dentalzemente ein plaquehemmender Effekt in den Druckschriften K. Larson, P. O. Glantz, Acta Odontol. Scand. 30 (1981), S. 79 und S. Minagi, et al., Infect. Immun. 48 (1985), S. 11 beschrieben.

Es ist weiterhin bekannt, daß Bakterienkolonien größtenteils nicht direkt auf dem Substrat angesiedelt sind, sondern vielmehr auf einer Pellikelschicht, die innerhalb kurzer Zeit im menschlichen Mund aus Speichelproteinen aufgebaut wird. Auf hydrophilen Oberflächen, beispielsweise auf natürlichen Zähnen oder Keramik, ist die Proteinadsorption reversibel, dagegen ist die Proteinadsorption auf unpolaren Oberflächen irreversibel, wodurch auch die Anhaftung von Mikroorganismen und somit letztendlich die Ausbildung einer bakteriellen Plaque begünstigt wird. Moderne Dentalcomposite zeichnen sich durch relativ hydrophobe Monomere, wie beispielsweise 2,2-Bis-[p-2-hydroxy-3-methacryloyloxypropoxy)phenyl]-propen (Bis-GMA) und 2,2,4-(2,4,4)-Trimethylhexamethylen-bis-(urethanethylmethylmethacrylat) (UDMA), aus, um die Wasseraufnahme und somit eine mögliche Quellung und die Anfärbbarkeit durch exogene Farbstoffe gering zu halten. Dies begünstigt allerdings andererseits die Neigung zu Akkumulation von Plaque. Gealterte und mineralisierte Plaque kann durch normales Zähneputzen nicht mehr entfernt werden und ist meistens durch Nahrungsbestandteile oder Tabakkonsum oder andere exogene Einflüsse gelblich bis bräunlich gefärbt, wodurch die betroffenen Oberflächen den an sie gestellten ästhetischen Ansprüchen nicht mehr genügen.

### Stand der Technik

Methoden zur Plaqueinhibierung an Zähnen oder dentalen Restaurationen sind aus dem Stand der Technik wohlbekannt. Diese Methoden lassen sich in drei Kategorien einteilen:
1. Beschichtung der Zähne oder dentalen Restauration mit Filmen
2. Beschichtung der Zähne oder dentalen Restauration mit antibakteriellen Filmen
3. Zusätze von antibakteriellen Agentien zu den Restaurationsmaterialien.

### ad 1.:

Silikonöle wurden aufgrund ihrer hydrophoben Natur als Zusatz zu Zahnpasten vorgeschlagen. Ihre Wirkung beruht auf dem Aufbau einer Grenzschicht zwischen zwei hydrophilen schichten, Zahn und Pellikel. Die Adhäsion und Retention an den Zahnoberflächen ist aber typischerweise gering.

Die US-A-5 078 988 (Lin et al.) offenbart Zahnpasten mit modifizierten Aminoalkylsilikonen, die eine hydrophobe Schicht auf den Zähnen erzeugen sollen, um bakterielle Anlagerungen zu vermindern.

Plaqueinhibierung für Zahnersatz auf Kunststoffbasis wird auch in der EP-A-0 575 535 (Essential Dental Systems Inc., 29.12. 1993) beschrieben. Nach dem Auftragen einer Masse, bestehend aus Lösungsmittel, flüchtiger Verbindung, Polyurethanharz und Polyfluorverbindung, auf den Zahnersatz wird die Beschichtung durch Luft- oder Wärmetrocknung fixiert.

Die PCT-Anmeldung WO-A-95/15740 (3M, 2.12.1994) beschreibt zur Verminderung der Plaqueanlagerung bei dentalen Werkstoffen ein mit der Oberfläche quervernetzbares Beschichtungsmittel aus copolymeren Einheiten. Prinzipiell wird die Oberfläche hydrophiliert und somit eine reversible Adhäsion der Bakterienkulturen ermöglicht.

E. Budtz-Joergensen und S. Kaaber, Scandinavian Journal of Dental Research, 94 (1986), S. 568-574, haben vorgeschlagen, den Zahnersatz mit quervernetzten Acrylpolymeren zu beschichten. Die Beschichtung wird photochemisch vorgenommen und ist daher unbequem und nur einmalig anwendbar.

Harvey et al. (US-A-5 192 362 vom 9.3.1993) verfolgenden den Ansatz, eine anionische Schicht, in diesem Falle aus Polysacchariden, aufzubringen und so die Adhäsion der Plaque zu vermindern.

### ad. 2.:

Die WO-A-91/13608 (Rolla et al) beschreibt Zahnpasten, die ein flüssiges Silikonöl und ein fettlösliches antibakterielles Mittel beinhalten, wobei die Wirkung durch die langsame Abgabe dieses antibakteriellen Mittels in den Speichel entfaltet wird.

Antibakterielle Filme zur Erhöhung der Lagerstabilität von Nahrungsmitteln sind aus JP-A-01186804-A (Dainippon Printing KK, 26.7.1989) bekannt. Diese Filme bestehen aus festen Zeolithpartikeln, die antibakteriell wirksame Metallionen beinhalten, und einem Oberflächenbehandlungsmittel, beide in Kunststoff dispergiert.

Dental einzusetzende Zubereitungen mit Amidobetainen oder Sulfobetainen als wirksame Bestandteile sind aus der DE-A-2 646 199 (Noxell Corp., 28.4.1977) bekannt und zeigen plaqueverhindernden Effekt.

Die Erfinder offenbaren in der EP-A-0 404 558 (Perio Prod. Ltd., Yissum Res. & Dev. Co., Petr Prod. Ltd., 27.12.1990) eine flüssige Zusammensetzung, welche Acrylpolymere, ein pharmazeutisches Produkt und ein Kontrollmittel für die Abgabe des Pharmazeutikums in den Mundraum, bevorzugt Quervernetzer, Polysaccharid, Lipid, Protein oder Aminosäure enthält und zur Verhinderung von Plaqueanlagerungen oder Behandlung von Infektionen im Mundraum dient.

### ad 3.:

Die EP-A-0 537 774 beschreibt dentale Compositematerialien, die antibakterielle einpolymerisierbare Monomere auf Basis quartärer Stickstoffverbindungen beinhalten.

Jusan P. Loyola-Rodriguez et al., Pediatric Dentistry 16 (1994), S. 345 ff. beschreiben einen kariesinhibierenden Einfluß von Glasionomerzementen, deren antibakterieller Effekt einer Fluoridabgabe von mindestens 140 ± 25 ppm und dem niedrigen pH-Wert vor der Abbindung des Zementes zugeschrieben wird.

Der Stand der Technik beschreibt somit als Mittel zur Plaqueinhibierung bei Zahnersatz antibakterielle und anionische Beschichtungen oder die Verwendung fluoridabgebender Zusätze. Neben dem beträchtlichen Zeitaufwand bei nachträglich aufzubringenden Beschichtungen und den daraus sich ergebenden Kosten für alle Beteiligten, kann aus Beschichtungen, die antibakterielle Wirkstoffe enthalten, wegen der Möglichkeit der Resistenzentwicklung bei pathogenen Oralkeimen, eine Gesundheitsgefährdung für Zahntechniker, Zahnarzt und Patient resultieren. Es besteht daher ein hoher Bedarf an per se plaquevermindernden Kunststoffmaterialien.

### Aufgabe und Lösung der Erfindung

Der vorliegenden Erfindung liegt die Aufgabe zugrunde, ein die Plaqueakkumulation verringerndes dentales Compositematerial zur Verwendung als dentales Restaurationsmaterial, insbesondere als Prothesen-, Provisorien-, Verblend- und Füllungswerkstoff sowie als Kunststoff für kieferorthopädische Geräte, künstliche Zähne, Stumpfaufbau, Versiegelung oder für Zemente bereitzustellen.

Die Aufgabe wird gelöst durch eine dentale Compositezusammensetzung auf der Basis von üblichen polymerisierbaren Monomeren, Initiatoren, Füllstoffen und anderen Hilfsstoffen, welche zusätzlich Salze von säurefunktionellen Polymeren mit ein- oder mehrwertigen Kationen, vorzugsweise ausgehärtete Glasionomerzemente, enthält, wobei die Salze als gemahlene Pulver vorliegen und mit der Compositerezeptur vermischt werden.

Es wurde überraschenderweise gefunden, daß die Zugabe von Salzen von säurefunktionellen Polymeren mit ein- oder mehrwertigen Kationen, insbesondere von ausgehärteten Glasionomerzementen, zu dentalem Compositematerial eine starke plaquehemmende Wirkung ergibt, obwohl im Gegensatz zu den aus dem Stand der Technik bekannten Methoden eine kaum nachzuweisende Menge Fluoridionen, nämlich weniger als 5 ppm, freigesetzt wird.

Überraschend ist darüber hinaus, daß die erfindungsgemäßen Compositematerialien nicht nur weniger Plaque anlagern, sondern daß gleichwohl gebildete Ablagerungen besonders leicht, beispielsweise durch konventionelles Putzen mit handelsüblichen Zahnbürsten, entfernbar sind.

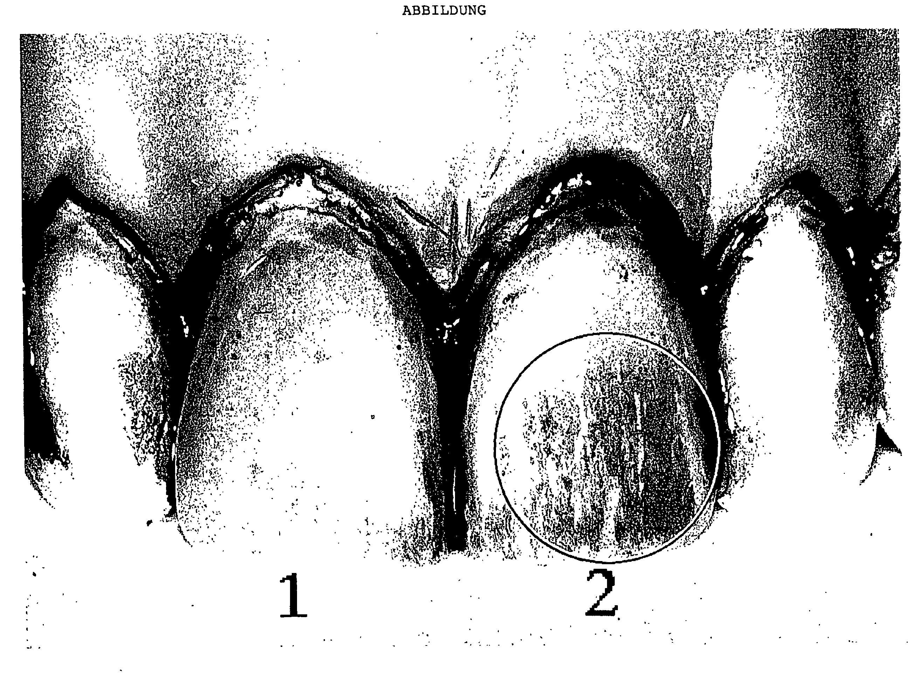

Die Abbildung zeigt die Totalprothese eines Patienten, bei der ein Zahn (Ziffer 1) mit erfindungsgemäßem Compositmaterial und ein Zahn (Ziffer 2) mit handelsüblichem Compositematerial behandelt wurde, nach sechswöchiger Tragedauer.

### Detaillierte Beschreibung der Erfindung

Die Erfindung betrifft dentale Compositezusammensetzungen, umfassend
(a) ein oder mehrere ethylenisch ungesättigte, polymerisierbare Monomere auf der Basis von mono-, di- oder polyfunktionellen (Meth)acrylaten,
(b) übliche Initiatoren und gegebenenfalls übliche Aktivatotoren,
(c) übliche Füllstoffe,
(d) gegebenenfalls Pigmente, röntgenopake Zusatzstoffe, Thixotropiehilfsmittel, Weichmacher und andere übliche Hilfsstoffe,
   welche dadurch gekennzeichnet sind, daß sie zusätzlich
(e) ein oder mehrere Salze von säurefunktionellen Polymeren mit ein- oder mehrwertigen Kationen, gegebenenfalls in Verbindung mit anorganischen Gläsern, d.h. in Form von ausgehärteten Glasionomerzementen, enthalten, wobei die Salze gemäß Komponente (e) als gemahlene Pulver vorliegen und mit der Compositerezeptur vermischt werden.

Die erfindungsgemäßen Compositezusammensetzungen enthalten die Komponenten (a) bis (e) in den folgenden bevorzugten Mengen:
(a) 4 bis 68,99 Gew.-%, vorzugsweise 45 bis 55 Gew.-%,
(b) 0,01 bis 3 Gew.-%, vorzugsweise 0,1 bis 2 Gew.-%,
(c) 5 bis 95 Gew.-%, vorzugsweise 20 bis 80 Gew.-%, noch bevorzugter 35 bis 70 Gew.-%,insbesondere 45 bis 55 Gew.-%,
(d) 0 bis 20 Gew.-%, vorzugsweise 0 bis 10 Gew.-%, und
(e) 1 bis 20 Gew.-%, vorzugsweise 2 bis 15 Gew.-%, insbesondere 3 bis 9 Gew.-%.

Sämtliche Prozentangaben sind jeweils auf das Gesamtgewicht der Compositezusammensetzung einschließlich aller Komponenten (a) bis (e) bezogen.

Als Komponente (a) kommen ethylenisch ungesättigte Monomere bzw. Polymere in Frage, z.B. monomere oder polymere Acrylate und Methacrylate. Hingewiesen sei diesbezüglich auf die in der DE-A-3 609 038 beschriebenen Massen, wobei auch die dort beschriebenen röntgenopaken Füllstoffe enthalten sein können. Als ethylenisch ungesättigte Monomere bzw. Polymere seien beispielsweise die monomeren und polymeren Acrylate und insbesondere Methacrylate hervorgehoben. Bei polymerisierbaren Dentalmassen verwendet man insbesondere oft die langkettigen Monomere der US-A-3 066 112 auf der Basis von Bisphenol-A und Glycidylmethacrylat oder deren durch Addition von Isocyanaten erhaltenen Derivate. Besonders geeignet sind auch die Acrylsäure- bzw. Methacrylsäureester ein- oder vorzugsweise mehrwertiger Alkohole, beispielsweise Triethylenglykoldimethacrylat und dgl. Besonders geeignet sind auch die in der DE-A-2 816 823 genannten Diacryl- und Dimethacrylsäureester des Bishydroxymethyltricyclo-[5.2.1.0^{2,6}]-decans. Verwendet werden können auch die Reaktionsprodukte aus Diisocyanaten und Hydroxyalkyl(meth)acrylaten, wie beispielsweise in der DE-A-2 312 559 beschrieben. Selbstverständlich können auch Gemische aus Monomeren bzw. hieraus hergestellte ungesättigte Polymere verwendet werden.

Als Photoinitiatoren der Komponente (b) können alle Substanzen eingesetzt werden, die nach Bestrahlen durch UV- oder sichtbarem Licht die Polymerisation auslösen, beispielsweise Benzoinalkylether, Benzilketale, Acylphosphinoxide oder aliphatische und aromatische 1,2-Diketonverbindungen, z.B. Campherchinon, wobei die Lichtpolymerisation durch Zusatz von Aktivatoren, wie tertiären Aminen oder organischen Phosphiten, in an sich bekannter Weise beschleunigt werden kann. Die US-A-3 541 068 und die DE-A-2 658 530 offenbaren dafür geeignete Aniline und Xylidine. Geeignete Initiatorsysteme zur Auslösung der Polymerisation über einen Redox-Mechanismus sind beispielsweise die Systeme Peroxid/Amin oder Peroxid/Barbitursäurederivate und dgl.. Bei Verwendung solcher Initiatorsysteme ist es zweckmäßig, einen Initiator (z.B. Peroxid) und eine Katalysatorkomponente (z.B. Amin) getrennt bereitzuhalten. Die beiden Komponenten werden dann kurz vor der Anwendung miteinander homogen vermischt. Geeignete Initiatorsysteme zur Auslösung der Polymerisation über einen thermischen Mechanismus sind Peroxide, beispielsweise Dibenzoylperoxid oder Dilauroylperoxid.

Die Füllstoffe der Komponente (c) haben vorzugsweise eine mittlere Kornverteilung von < 20 µm, insbesondere < 5 µm und ganz besonders bevorzugt < 1,5 µm, sowie eine obere Korngröße von 70 µm, vorzugsweise 25 µm und insbesondere < 5 µm. Anorganische Füllstoffe können beispielsweise Quarz, gemahlene Gläser, Kieselgele sowie pyrogene Kieselsäuren oder deren Granulate sein. Auch können röntgenopake Füllstoffe, zumindest teilweise, mit eingesetzt werden. Diese können zum einen röntgenopake Gläser sein, also Gläser, welche z.B. Strontium, Barium oder Lanthan enthalten. Zum besseren Einbau in die Polymermatrix ist es von Vorteil, die anorganischen Füllstoffe zu hydrophobieren. Übliche Hydrophobierungsmittel sind Silane, beispielsweise Trimethoxymethacryloyloxypropylsilan. Die Menge des eingesetzten Silans beträgt üblicherweise 0,5 bis 10 Gew.-%, bezogen auf anorganische Füllstoffe, bevorzugt 1 bis 6 Gew.-%, ganz besonders bevorzugt 2 bis 5 Gew.-%. Als Füllstoffe eignen sich auch bereits fertig pigmentierte Polymethylmethacrylatperlen oder andere pulverisierte organische Polymerisate.

Komponente (d) kann ein röntgenopaker Zusatz, wie beispielsweise Yttriumfluorid, Strontiumhexafluorozirkonat oder Fluoride der Selten-Erdmetalle, sein. Zur Erhöhung der Flexibilität der Massen kann es auch vorteilhaft sein, lösliche organische Polymere einzusetzen. Geeignet sind z.B. Polyvinylacetat sowie die Copolymeren auf Basis Vinylchlorid/Vinylacetat und dgl.. Gut geeignet als zusätzliche Weichmacher sind beispielsweise Dibutyl-, Dioctyl- und Dinonylphthalate.

Erfindungsgemäß wird zu den Bestandteilen (a) bis (d) zur Plaqueinhibierung die Komponente (e) in Form eines oder mehrerer Salze von säurefunktionellen Polymeren mit mehrwertigen Kationen, insbesondere ausgehärtete Glasionomerzemente, hinzugegeben. Diese werden bevorzugt auf Korngrößen unterhalb 20 µm gemahlen. Die entstandenen Pulver werden mit Compositerezepturen vermischt und diese wie üblich verarbeitet. Die eingesetzten Salze können auch auf anderen Wegen hergestellt werden, beispielsweise durch Sprühtrocknung.

Der Kationenanteil der Salze von säurefunktionellen Polymeren mit mehrwertigen Kationen kann aus der Gruppe:
I., II. und III. Hauptgruppe, II. und III. Nebengruppe sowie der Lanthaniden in Form der entsprechenden Ionen entnommen sein. Bevorzugte Kationen der I. Hauptgruppe sind Li, Na und K, vorzugsweise Na. Bevorzugte Kationen der II. Hauptgruppe sind Ca, Mg, Sr, Ba, wobei insbesondere Calcium in Betracht kommt. Als Kationen der III. Hauptgruppe kommen Al, Ga und In in Frage, insbesondere Al und In, wobei Aluminium besonders bevorzugt ist.

Aus der II. Nebengruppe eignet sich insbesondere Zn als Kation, und aus der III. Nebengruppe können Sc, Y und La herangezogen werden, insbesondere Y und La. Von den Lanthaniden werden Ce und Yb bevorzugt.

Als einzusetzende polymere Polysäure eignen sich Polycarbonsäuren, z.B. Polymaleinsäure, Polyacrylsäure, Polyitaconsäure sowie Gemsiche davon oder Copolymere, insbesondere die aus EP-A-0 024 056 bekannten Maleinsäure/Acrylsäure-Copolymeren und/oder Acrylsäure/Itaconsäure-Copolymeren sein. Das mittlere Molekulargewicht der einzusetzenden Polycarbonsäure beträgt mehr als 500. Vorteilhaft ist ein mittleres Molekulargewicht von 1.000 bis 20.000, besonders bevorzugt sind solche von 3.000 bis 10.000. Als polymere Polysäure eignen sich auch Polyphosphonsäuren, z.B. Polyvinylphosphonsäure. Diese Polyphosphonsäuren können die oben genannten Polycarbonsäuren ganz oder teilweise ersetzen. In Wasser nicht lösliche Verbindungen der Kationen (z.B. ZnO) werden in der Säurelösung suspendiert, die ausfallenden Salze werden eventuell erhitzt, um die Reaktion zu vervollständigen. Anschließend wird nach bekannten Methoden aufgearbeitet und getrocknet.

Glasionomerzemente bestehen im allgemeinen aus:
(a) einem Aluminiumfluorosilikatglas oder einem Aluminiumsilikatglas
(b) mindestens einer polymeren Polysäure mit einem mittleren Molekulargewicht > 500
(c) Wasser
(d) gegebenenfalls einem chelatbildenden Mittel.

Als Bestandteil (a) können die in der DE-A-2 061 513 und in der EP-A-0 023 013 beschriebenen Calciumaluminiumfluorosilikatgläser eingesetzt werden.

Aluminiumsilikatgläser bzw. Aluminiumfluorosilikatgläser bestehen neben Sauerstoff beispielsweise aus:

| **Bestandteil** | **Berechnet als** | **Gew.-%** |
|---|---|---|
| Si | SiO₂ | 2 - 60 |
| Al | Al₂O₃ | 10 - 50 |
| Ca | Ca0 | 0 - 40 |
| Sr | SrO | 0 - 40 |
| F | F | 0 - 40 |
| Na | Na₂O | 0 - 10 |
| P | P₂O₅ | 0 - 10 |

wobei mindestens 1 Gew.-% CaO und/oder SrO enthalten sein müssen und insgesamt 0 bis 20 Gew.-%, berechnet als Oxide, B, Bi, Zn, Mg, Sn, Ti, Zr, La oder andere dreiwertige Lanthanoiden, K, W, Ge sowie weitere Zusätze, welche die Eigenschaften nicht beeinträchtigen und physiologisch unbedenklich sind. Durch Zusatz von 10 bis 20 Gew.-% La₂O₃ können die Gläser röntgensichtbar gemacht werden.

Vorteilhaft bestehen die fluoridhaltigen Pulverteilchen aus:

| **Bestandteil** | **Berechnet als** | **Gew.-%** |
|---|---|---|
| si | SiO₂ | 25 - 50 |
| Al | Al₂O₃ | 10 - 40 |
| Ca | CaO | 0 - 35 |
| Sr | SrO | 0 - 35 |
| F | F | 10 - 30 |
| Na | Na₂O | 0 - 8 |
| P | P₂O₅ | 1 - 10 |

wobei mindestens 10 Gew.-% Ca (berechnet als CaO) und/oder Sr (berechnet als SrO) enthalten sein müssen und 0 bis 10 Gew.-% B₂O₃, Bi₂O₃, ZnO, MgO, SnO₂, TiO₂, ZrO₂, La₂O₃ oder andere Oxide dreiwertiger Lanthanoide, K₂O, WO₃, GeO₂ sowie weitere Zusätze, welche die Eigenschaften nicht beeinträchtigen und physiologisch unbedenklich sind.

Besonders bevorzugt verwendete fluoridhaltige Pulver enthalten:

| **Bestandteil** | **Berechnet als** | **Gew.-%** |
|---|---|---|
| Si | SiO₂ | 25 - 45 |
| Al | Al₂O₃ | 20 - 40 |
| Ca | Ca0 | 10 - 30 |
| F | F | 10 - 30 |
| Na | Na₂O | 1 - 8 |
| P | P₂O₅ | 1 - 10 |

Die zu verwendenden Glaspulver als Bestandteil eines herkömmlichen Glasionomerzements weisen eine durchschnittliche Korngröße (Gewichtsmittel) von wenigstens 1 µm und bevorzugt mindestens 3 µm auf. Die durchschnittliche Korngröße (Gewichtsmittel) beträgt 1 bis 20 µm, bevorzugt 3 bis 15 µm und besonders bevorzugt 3 bis 10 µm. Die Teilchen haben eine maximale Korngröße von 100 µm, vorzugsweise 60 µm, insbesondere 20 µm.

Die so erhaltenen Pulver werden dann gegebenenfalls einer Oberflächenbehandlung gemäß der EP-A-0 023 013 unterzogen. Hierzu werden die Glaspulver mit Säure oberflächlich behandelt, vorzugsweise bei Raumtemperatur. Dabei werden saure Gruppen enthaltende Substanzen eingesetzt, z.B. Salzsäure, Schwefelsäure, Salpetersäure, Essigsäure, Propionsäure oder Perchlorsäure, die lösliche Calciumsalze bzw. Strontiumsalze bilden. Die Säuren werden in einer Konzentration von 0,01 bis 10 Gew.-%, vorzugsweise 0,05 bis 3 Gew.-%, eingesetzt. Nach der entsprechenden Reaktionszeit werden die Pulver von der Lösung separiert und gründlich ausgewaschen, so daß sich praktisch keine löslichen Calcium- oder Strontiumsalze mehr auf der Oberfläche der Pulverteilchen befinden.

Nicht fluoridhaltige Pulver können durch einen zusätzlichen, dem ehemaligen Fluoridanteil entsprechenden Anteil von 10 bis 30 Gew.-% Oxidmischung erhalten werden.

Die als Bestandteil (b) einzusetzenden polymeren Polysäuren können Polycarbonsäuren, z.B. Polymaleinsäure, Polyacrylsäure, Polyitaconsäure sowie Gemische davon oder Copolymere, insbesondere die aus der EP-A-0 024 056 bekannten MaleinsäureAcrylsäure-Copolymeren und/oder Acrylsäure-Itaconsäure-Copolymeren sein. Das mittlere Molekulargewicht der einzusetzenden Polycarbonsäure beträgt mehr als 500. Vorteilhaft ist ein mittleres Molekulargewicht von 1.000 bis 20.000, besonders bevorzugt sind 3.000 bis 10.000. Die Polysäure wird vorzugsweise in Konzentrationen von 5 bis 50 Gew.-% bezogen auf Bestandteil (a) eingesetzt. Als polymere Polysäure eignen sich auch Polyphosphonsäuren, z.B. Polyvinylphosphonsäure. Diese Polyphosphonsäuren können die oben genannten Polycarbonsäuren ganz oder teilweise ersetzen.

Der Bestandteil (c), das Wasser, wird in Mengen von 5 bis 70 Gew.-%, vorzugsweise 15 bis 40 Gew.-%, bezogen auf das Gesamtgewicht, eingesetzt.

Als Bestandteil (d) kann ein chelatbildendes Mittel, wie es in der DE-A-2 319 715 beschrieben ist, enthalten sein. Vorzugsweise wird als Chelatbildner Weinsäure eingesetzt. Die Chelatbildner können in Konzentrationen von 0,1 bis 10, vorzugsweise 3 bis 8 Gew.-%, bezogen auf die Gesamtmasse, eingesetzt werden.

Um hohe Lagerstabilität vor der Anwendung zu erhalten, empfiehlt sich ein Zusatz von Konservierungsstoffen, z.B. Benzoesäure, insbesondere zur trockenen Polysäure.

Zusätze zur Einstellung der Viskosität (z.B. pyrogene Kieselsäure) sind möglich. Geeignete Konzentrationen sind 0,1 bis 10, vorzugsweise 1 bis 5 Gew.-%, bezogen auf die Gesamtmasse.

Die erfindungsgemäßen Compositezusammensetzungen eigenen sich insbesondere als Prothesen-, Provisorien-, Verblend- und Füllungswerkstoffe sowie als Kunststoffe für kieferorthopädische Geräte, künstliche Zähne, Stumpfaufbau, zur Versiegelung oder als Zemente.

### Beispiele

Die Erfindung wird durch nachfolgende Versuchsbeispiele weiter erläutert, wobei der Inhalt der Versuchsbeschreibungen nur exemplarischen Charakter hat und die Erfindung keinesfalls auf diese Beispiele beschränkt sein soll.

### Beispiel 1

Die Erfindung wird durch Einteilung der nachfolgend beschriebenen Versuchsbeispiele in drei Gruppen besonders einfach verständlich. Die Versuchsgruppe A mit den Versuchen 1 bis 7 dient der Herstellung feinst gemahlenen Pulvers aus abgebundenen Glasionomerzementen (1 bis 5) bzw. feinst gemahlenem Pulver aus Salzen säurefunktioneller Polymeren mit mehrwertigen Kationen (6 bis 7). Die Versuchsgruppe B, bestehend aus Versuch 8, beschreibt eine Compositerezeptur wie sie aus dem Stand der Technik bekannt ist. Bei der Versuchsgruppe C, bestehend aus den Versuchen 9 bis 17, handelt es sich um Ausgestaltungen der Erfindung, wobei jeweils dentales Compositematerial aus Versuchsgruppe B mit den Pulvern aus Versuchsgruppe A vermischt und zur Aushärtung gebracht wird. Anschließend werden nach den im folgenden beschriebenen Verfahren Plaqueanlagerungen in vitro und in vivo, sowie die Fluoridfreisetzung im wäßrigen Milieu untersucht. Das Zeta-Potential an der Oberfläche der ausgehärteten Compositematerialien wird durch die Zugabe der erfindungsgemäßen Polysalze negativer; bei vergleichbaren Rezepturen korreliert dieser Effekt mit dem Ausmaß der Plaquereduktion.

Die jeweils eingesetzten Glaspulverkomponenten dienen als Quelle für mehrwertige Kationen, die eingesetzten Flüssigkeiten beinhalten säurefunktionelles Polymer.

### Versuchsgruppe A

Bei den nachstehend beschriebenen Versuchen werden handelsübliche Glaspulver und Flüssigkeiten eingesetzt:
- Glaspulver 1:: Ketac Fil Glaspulver (Fa. Espe)
- Glaspulver 2:: Fuji II Glaspulver (Fa. GC)
- Glaspulver 3:: Chemfil Superior Glaspulver + Polymersäure (Fa. Dentsply)
- Flüssigkeit 1:: Ketac Fil Flüssigkeit (Fa. Espe) (Polymersäure + Wasser)
- Flüssigkeit 2:: Durelon Flüssigkeit (Fa. Espe) (Polymersäure + Wasser)
- Flüssigkeit 3:: Fuji II Flüssigkeit (Fa. GC) (Polymersäure + Wasser)

### Versuch 1

Glaspulver 1 wird mit Flüssigkeit 1 im Verhältnis 2:1 vermischt. Der abgebundene Zement wird anschließend auf eine Korngröße < 20 µm vermahlen.

### Versuch 2

Glaspulver 1 wird mit Flüssigkeit 2 im Verhältnis 2:1 vermischt. Der abgebundene Zement wird anschließend auf eine Korngröße < 20 µm vermahlen.

### Versuch 3

Glaspulver 1 wird mit 20 % Polyvinylphosphonsäure (PVP) im Verhältnis 2:1 vermischt. Der abgebundene Zement wird anschließend auf eine Korngröße < 20 µm vermahlen.

### Versuch 4

Glaspulver 2 wird mit Flüssigkeit 3 im Verhältnis 2:1 vermischt. Der abgebundene Zement wird anschließend auf eine Korngröße < 20 µm vermahlen.

### Versuch 5

Glaspulver 3 wird mit destilliertem Wasser im Verhältnis 7:1 vermischt. Der abgebundene Zement wird anschließend auf eine Korngröße < 20 µm vermahlen.

### Versuch 6

Aluminiumoxid C (Fa. Degussa) wird mit Flüssigkeit 1 im Verhältnis 1:1 vermischt. Der gebildete salzartige Festkörper wird anschließend auf eine Korngröße < 20 µm vermahlen.

### Versuch 7

Aluminiumhydroxidphosphat (Fa. Chemische Fabrik Budenheim) wird mit Flüssigkeit 1 im Verhältnis 2:1 vermischt. Der gebildete salzartige Festkörper wird anschließend auf eine Korngröße < 20 µm vermahlen.

### Versuchsgruppe B

### Versuch 8

Aus

| | |
|---|---|
| 29 g | Bis-(acryloxymethyl)-tricyclo[5.2.1.0^{2,6}]-decan |
| 15 g | 2,2,4-(2,4,4)-Trimethylhexamethylen-bis-(urethanethylmethacrylat) |
| 5 g | 1,12-Dodecandioldimethacrylat |
| 40,4 g | silanisiertem Ba-Al-Borosilikatglas d₅₀ = 0,7 µm |
| 5 g | Calciumfluorid |
| 4,5 g | silanisiertem Aerosil OX 50 (Fa. Degussa) |
| 0,9 g | N,N-Dimethylaminoethylmethacrylat |
| 0,1 g | Campherchinon |
| 0,1 g | Titandioxid und Eisenpigmenten |

wird eine homogene Paste geknetet.

### Versuchsgruppe C

### Versuch 9

In der Rezeptur aus Versuch 8 werden anstelle des Calciumfluorids 5 g des Zementpulvers aus Versuch 1 eingesetzt.

### Versuch 10

In der Rezeptur aus Versuch 8 werden anstelle des Calciumfluorids 2 g silanisiertes Ba-Al-Borosilikatglas d₅₀ = 0,7 µm und 3 g des Zementpulvers aus Versuch 1 eingesetzt.

### Versuch 11

In der Rezeptur aus Versuch 8 werden anstelle des Calciumfluorids 8 g Zementpulver aus Versuch 1 und lediglich 37,4 g silanisiertes Ba-Al-Borosilikatglas d₅₀ = 0,7 µm eingesetzt.

### Versuch 12

In der Rezeptur aus Versuch 8 werden anstelle des Calciumfluorids 5 g des Zementpulvers aus Versuch 2 eingesetzt.

### Versuch 13

In der Rezeptur aus Versuch 8 werden anstelle des Calciumfluorids 5 g des Zementpulvers aus Versuch 3 eingesetzt.

### Versuch 14

In der Rezeptur aus Versuch 8 werden anstelle des Calciumfluorids 5 g des Zementpulvers aus Versuch 4 eingesetzt.

### Versuch 15

In der Rezeptur aus Versuch 8 werden anstelle des Calciumfluorids 5 g des Zementpulvers aus Versuch 5 eingesetzt.

### Versuch 16

In der Rezeptur aus Versuch 8 werden anstelle des Calciumfluorids 2 g silanisiertes Ba-Al-Borosilikatglas d₅₀ = 0,7 µm und 3 g des Salzes aus Versuch 6 eingesetzt.

### Versuch 17

In der Rezeptur aus Versuch 8 werden anstelle des Calciumfluorids 5 g des Salzes aus Versuch 7 eingesetzt.

### Durchführung des Plaquetests

Das Ausmaß der Plaqueanlagerung wurde in vitro durch die nachfolgend beschriebene Prüfmethode nachgewiesen.
1. Herstellung von Compositeplättchen mit einem Durchmesser von 10 mm und einer Höhe von 1,5 mm.
2. Standardisierte maschinelle Politur der Plättchen aus 1. mit zuletzt 1 µm Diamantsuspension.
3. Anzucht der Testkeime: Streptococcus sanguis, biotype 1, DSM 20068 und Streptococcus mutans, ATCC 25175, DSM 20523 (DSM = Deutsche Sammlung von Mikroorganismen) werden jeweils in einer Flasche mit Caso-Bouillon angezüchtet, indem die beimpfte Bouillon bei 30-35°C inkubiert wird, bis eine starke Trübung vorliegt. Zur Anzucht der Testkeime werden aus dieser Bouillon Platten aus Caso-Bouillon + 0,5 % Agar mit jeweils 0,5 ml beimpft und 2-3 Tage bei 30-35°C bebrütet.
4. Herstellung der Keimsuspension: Mit Hilfe einer Impföse werden die Keime von bewachsenen Platten in Ringerlösung suspendiert, bis eine starke Trübung vorliegt. Die Keimsuspension wird anschließend 10 Minuten im Ultraschallbad behandelt und 1:10 mit synthetischer Speichellösung (Shellis R.P.; Archives of Oral Biology; 23, (1978), S. 485-489 modifiziert nach Glenister D.A., Salamon K. E., et al.; Microbial Ecology in Health and Disease; 1, (1988), S. 31-38) verdünnt und homogenisiert. Die fertige Keimsuspension enthält Streptococcus mutans (ca. 10⁷-10⁸ KBE/ml) und Streptococcus sanguis (ca. 10⁶-10⁷ KBE/ml).
5. Testansatz: Die Prüfplättchen werden in 70 %igem Ethanol desinfiziert. Bei jedem Testansatz werden Referenzplättchen aus Visio Gem Verblendcomposite (Fa. Espe) zum Vergleich mitgeführt. Nach dem Trocknen werden alle Plättchen, die zu der gleichen Probe gehören, mit Hilfe einer sterilen Pinzette zusammen in eine sterile 100 ml-Flasche überführt und mit 10 ml der Keimsuspension versetzt, wobei die Suspension jedesmal vor Entnahme durch Schütteln gut homogenisiert werden muß. Danach erfolgt eine Keimzahlkontrolle der Keimsuspension. Anschließend werden die Proben für 24 Stunden bei 37°C und 100 rpm im Schüttelwasserbad inkubiert. Eine weitere Probe der Keimsuspension wird, gemeinsam mit den Prüfplättchen, ebenfalls für 24 Stunden inkubiert und anschließend erneut auf ihre Keimzahl überprüft. Nach der Inkubation werden die Prüfplättchen, nach der Dekantierung der Keimsuspension, in ein Becherglas mit 100 ml Ringerlösung überführt. Alle zu einer Probe gehörenden Prüfplättchen werden in das gleiche Becherglas gegeben. Anschließend wird das Becherglas geschüttelt und die Prüfplättchen mit Hilfe einer sterilen Pinzette in ein zweites Becherglas überführt. Dieser Vorgang wird dann noch dreimal wiederholt. Aus dem fünften Becherglas werden die Plättchen einzeln in Reagenzgläser mit Ringerlösung mit Zusatz von 1 % Tween 20 gegeben. Diese Röhrchen werden dann 10 Minuten im Ultraschallbad behandelt. Danach werden die Proben gründlich gemischt und eine Keimzahlbestimmung mittels Gußplattenmethode durchgeführt. Als Nährmedium wird Caso-Bouillon mit 0,5 % Hefeextrakt und 1,5 % Agar verwendet. Die Platten werden nach dem Erkalten 5 Tage bei 30-35°C bebrütet. Gewachsene Kolonien werden anschließend ausgezählt und auf die gesamte Menge an Lösung, mit der die Plättchen im Ultraschallbad behandelt wurden, hochgerechnet (= KBE/Prüfplättchen). Pro Versuchsreihe werden mindestens drei Versuche mit jeweils mindestens drei Prüfplättchen durchgeführt.
6. Berechnung: Es werden nur solche Versuche gewertet, bei denen die relative Standardabweichung innerhalb einer Meßreihe < 20 % beträgt. Anschließend wird der Mittelwert aus den drei Versuchsreihen gebildet.

Zusätzlich zu den beschriebenen Ausführungsbeispielen wurden noch die handelsüblichen Compositematerialien Artglass (Fa. Kulzer) und Solidex (Fa. Shofu) in die Untersuchung miteinbezogen.

### Versuchsergebnisse

Die folgende Tabelle gibt eine Übersicht über die Versuchsergebnisse in Prozent relativ zu den Referenzplättchen aus Visio Gem:

| **Material** | **Streptococcus mutans** | **Streptococcus sanguis** |
|---|---|---|
| | **Stand der Technik** | |
| Artglass | 91,5 ⁺/₋ 4 | 96,7 ⁺/₋ 4,4 |
| Solidex | 107,7 ⁺/₋ 4,8 | 92,6 ⁺/₋ 5,4 |
| Versuch 8 | 97,7 ⁺/₋ 6,4 | 73,5 ⁺/₋ 7,4 |

| | **Erfindung** | |
|---|---|---|
| Versuch 9 | 57,8 ⁺/_ 7,3 | 28,9 ⁺/_ 11,8 |
| Versuch 10 | 64,8 ⁺/_ 8,1 | 44,1 ⁺/_ 14,2 |
| Versuch 11 | 37,2 ⁺/_ 6,0 | 19,3 ⁺/_ 3,6 |
| Versuch 12 | 63 ⁺/_ 8,1 | 33,0 ⁺/_ 6,3 |
| Versuch 13 | 22,9 ⁺/_ 8,5 | 20,7 ⁺/_ 7,4 |
| Versuch 14 | 86,6 ⁺/_ 17,1 | 67,0 ⁺/_ 5,1 |
| Versuch 15 | 64,9 ⁺/_ 15,9 | 42,9 ⁺/_ 13,1 |
| Versuch 16 | 73,5 ⁺/_ 7,4 | 40,2 ⁺/_ 5,3 |
| Versuch 17 | 39,6 ⁺/_ 4,6 | 43,3 ⁺/_ 4,5 |

### Beispiel 2

Der erfindungsgemäße Effekt der Verminderung der Plaqueanlagerung wurde überdies in vivo nach folgender Prüfmethode nachgewiesen: Die Totalprothese eines Probanden wurde an den Kunststoffzähnen "11" und "12" zurückgeschliffen. Ein Zahn wurde mit einem handelsüblichen Kunststoffverblendmaterial (Visio Gem, Fa. Espe), der andere mit dem erfindungsgemäßen Compositematerial aus Versuch 9 verblendet. Nach sechswöchiger Tragedauer ohne spezielle Hygieneinstruktionen erfolgte die Untersuchung der Prothese. Die während der Tragezeit angelagerte Plaque wurde mit Anfärbelösung "plaque check DS 2" (Fa. Hu-Friedy) deutlich rot gefärbt, wodurch eine digitale Erfassung und Auswertung ermöglicht wurde. Auf der Abbildung ist der erfindungsgemäße Effekt leicht zu erkennen. Die Abbildung zeigt die Totalprothese des Probanden, bei dem ein Zahn (bezeichnet mit der Ziffer 1) mit erfindungsgemäßen Compositematerial aus Versuch 9 verblendet wurde. Der mit Ziffer 2 bezeichnete Zahn wurde mit dem handelsüblichen Material (Visio Gem, Fa. Espe) verblendet. Die in dieser Schwarz-Weiß-Abbildung gezeigten dunklen Stellen, entsprechen den durch die erwähnte Plaqueanfärbelösung rot gefärbten Plaqueanlagerungen. Eine digitale Auswertung ergab zwischen Zahn 1 und Zahn 2 einen Unterschied in der Plaqueanlagerung von ca. 67 %.

### Beispiel 3

Der erfindungsgemäße Effekt der Verminderung der Plaqueanlagerung wurde ferner über das Zetapotential nach C. Werner, H.-J. Jacobasch, G. Reichelt, J. Biomat. Sci. Polymet. Edn., 7 (1995), No. 1, 61-76 nachgewiesen. N. Satou et al. veröffentlichten im Journal of Material Science (1996), 749-752 die Theorie, daß die Anlagerung von Bakterien durch negative Oberflächenpotentiale verhindert werden kann. Zwischen dem Zeta-Potential und der Anzahl der Streptococcus mutans, Streptococcus sanguis und Streptococcus sobrinus Kolonien auf einer Oberfläche besteht danach ein direkter Zusammenhang.

Jeweils zwei Prüfkörper der Dimension 20 x 10 x 1,5 mm mit einer auf 1 µm polierten Fläche werden der Meßprozedur unterzogen, zusätzlich zu den beschriebenen Ausführungsbeispielen wude noch das handelsübliche Compositematerial Visio Gem (Fa. Espe) in die Untersuchung miteinbezogen.

Es wurden folgende Ergebnisse erhalten:

| **Material** | **ξ [mV] bei pH = 7** |
|---|---|
| Visio Gem | -40 |
| Versuch 8 von Beispiel 1 | -44 |
| Versuch 9 von Beispiel 1 | -77 |
| Versuch 10 von Beispiel 1 | -60 |
| Versuch 11 von Beispiel 1 | -80 |

Es ist deutlich ersichtlich, daß mit zunehmendem Gehalt des Polysalzes bei ansonsten vergleichbaren Zusammensetzungen das Zeta-Potential negativer wird.

### Beispiel 4

Zum Nachweis, daß der erfindungsgemäße Effekt im Gegensatz zu plaqueinhibierenden Materialien aus dem Stand der Technik ohne bedeutende Fluoridionenabgabe auftritt, wurde bei den Materialien der Versuchsgruppe C, die gemahlene Glasionomerzemente aus fluoridhaltigen Gläsern enthalten, die Fluoridionenfreisetzung gemessen. Hierzu wurden zwei Probekörper mit 15 mm Durchmesser und 1,5 mm Höhe frei hängend in 50 ml destilliertem Wasser bei 36°C gelagert und nach dem jeweiligen Zeitraum die Fluoridionenkonzentration mittels einer ionensensitiven Elektrode gemessen. Nach jeder Messung erfolgte ein Wechsel des Wassers. Die Erfassungsgrenze der angewandten Methode liegt bei 0,1 ppm Fluorid.

| **Material von Versuch Nr. von Beispiel 1** | **9** | **10** | **11** | **12** | **13** | **14** | **15** |
|---|---|---|---|---|---|---|---|
| Fluoridkonzentration nach 1d [ppm] | <0,1 | <0,1 | 0,2 | <0,1 | <0,1 | <0,1 | 0,1 |
| Fluoridkonzentration nach 7d [ppm] | 0,1 | <0,1 | 0,1 | <0,1 | 0,1 | 0,2 | 0,2 |

## Patentansprüche

1. Dentale Compositezusammensetzung, umfassend
(a) ein oder mehrere ethylenisch ungesättigte, polymerisierbare Monomere auf der Basis von mono-, di- oder polyfunktionellen (Meth)acrylaten,
(b) übliche Initiatoren und gegebenenfalls.übliche Aktivatoren,
(c) übliche Füllstoffe,
(d) gegebenenfalls Pigmente, röntgenopake Zusatzstoffe, Thixotropiehilfsmittel, Weichmacher und andere übliche Hilfsstoffe,
**dadurch gekennzeichnet, daß** sie zusätzlich
(e) ein oder mehrere Salze von säurefunktionellen Polymeren mit ein- oder mehrwertigen Kationen, gegebenenfalls in Verbindung mit anorganischen Gläsern, enthält, wobei die Salze gemäß Komponente (e) als gemahlene Pulver vorliegen und mit der Compositerezeptur vermischt werden.

2. Compositezusammensetzung nach Anspruch 1, **dadurch gekennzeichnet, daß** die Komponente (e) ein Na-, Ca-, Al-, Zn-, Y- und/oder La-Salz einer polymeren Carbonsäure, ausgewählt aus der Gruppe bestehend aus Polymaleinsäure, Polyacrylsäure, Polyitaconsäure, Maleinsäure/Acrylsäure-Copolymeren und/oder Acrylsäure/Itaconsäure-Copolymeren, oder einer polymeren Phosphonsäure ist.

3. Compositezusammensetzung nach Anspruch 1, **dadurch gekennzeichnet, daß** die Komponente (e) ein ausgehärteter Glasionomerzement, vorzugsweise auf der Basis üblicher Calcium- oder Strontiumaluminiumfluorosilikatgläser, ist.

4. Compositezusammensetzung nach den Ansprüchen 1 bis 3, **dadurch gekennzeichnet, daß** die Komponente (e) auf eine kleine Korngröße von vorzugsweise unter 20 µm gemahlen worden ist.

5. Compositezusammensetzung nach den Ansprüchen 1 bis 4, **dadurch gekennzeichnet, daß** die Komponente (e) in einer Menge von 1 bis 20 Gew.-%, bezogen auf das Gesamtgewicht der Zusammensetzung, vorhanden ist.

6. Verwendung der Compositezusammensetzung nach den Ansprüchen 1 bis 5 zur Herstellung von Prothesen-, Provisorien-, Verblend- oder Füllungswerkstoffen oder zur Herstellung von Kunststoffen für kieferorthopädische Geräte, künstliche Zähne, Stumpfaufbauten oder Zementen.

7. Verwendung von Salzen von säurefunktionellen Polymeren mit ein- oder mehrwertigen Kationen, insbesondere von ausgehärteten Glasionomerzementen, nach einem der Ansprüche 1 bis 5, zur Herstellung von dentalen Compositezusammensetzungen mit plaqueinhibierendem Zusatz.

8. Compositezusammensetzungen nach einem der Ansprüche 1 bis 5, enthaltend die Komponenten (a) bis (e) in den folgenden Mengen:
(a) 4 bis 68,99 Gew.-%, vorzugsweise 45 bis 55 Gew.-%,
(b) 0,01 bis 3 Gew.-%, vorzugsweise 0,1 bis 2 Gew.-%,
(c) 5 bis 95 Gew.-%, vorzugsweise 20 bis 80 Gew.-%, noch bevorzugter 35 bis 70 Gew.-%,insbesondere 45 bis 55 Gew.-%,
(d) 0 bis 20 Gew.-%, vorzugsweise 0 bis 10 Gew.-%, und
(e) 1 bis 20 Gew.-%, vorzugsweise 2 bis 15 Gew.-%, insbesondere 3 bis 9 Gew.-%.

9. Compositezusammensetzung nach einem der Ansprüche 1 bis 5 oder 8, **dadurch gekennzeichnet, dass** die Menge an freisetzbaren Fluoridionen weniger als 5 ppm beträgt.

10. Compositezusammensetzung nach einem der Ansprüche 1 bis 5, 8 oder 9, **dadurch gekennzeichnet, dass** die säurefunktionellen Polymere gewählt sind aus Polymaleinsäure, Polyacrylsäure, Polyitaconsäure, Gemische davon, Maleinsäure/Acrylsäure-Copolymere, Acrylsäure/Itaconsäure-Copolymere und Polyphosphonsäure.

## Claims

1. Dental composite composition, comprising
(a) one or more ethylenically unsaturated, polymerizable monomers based on mono-, di- or polyfunctional (meth)acrylates,
(b) customary initiators and optionally customary activators,
(c) customary fillers,
(d) optionally pigments, X-ray-opaque additives, thixotropic auxiliaries, plasticizers and other customary auxiliaries,
**characterized in that** it additionally
(e) contains one or more salts of acid-functional polymers with mono- or polyvalent cations, optionally in combination with inorganic glasses, the salts according to component (e) being present as ground powders and being mixed with the composite formulation.

2. Composite composition according to Claim 1, **characterized in that** component (e) is an Na, Ca, Al, Zn, Y and/or La salt of a polymeric carboxylic acid, selected from the group consisting of polymaleic acid, polyacrylic acid, polyitaconic acid, maleic acid/acrylic acid copolymers and/or acrylic acid/itaconic acid copolymers, or a polymeric phosphonic acid.

3. Composite composition according to Claim 1, **characterized in that** component (e) is a cured glass ionomer cement, preferably based on customary calcium or strontium aluminium fluorosilicate glasses.

4. Composite composition according to Claims 1 to 3, **characterized in that** component (e) has been ground to a small particle size of preferably below 20 µm.

5. Composite composition according to Claims 1 to 4, **characterized in that** component (e) is present in an amount of 1 to 20% by weight, based on the total weight of the composition.

6. Use of the composite composition according to Claims 1 to 5 for the preparation of prosthetic, temporary, veneer or filling materials or for the preparation of plastics for orthodontic apparatuses, false teeth, stump build-ups or cements.

7. Use of salts of acid-functional polymers with mono- or polyvalent cations, in particular of cured glass ionomer cements, according to one of Claims 1 to 5, for the preparation of dental composite compositions containing a plaque-inhibiting additive.

8. Composite compositions according to one of Claims 1 to 5 comprising components (a) to (e) in the following amounts:
(a) 4 to 68.99% by weight, preferably 45 to 55% by weight,
(b) 0.01 to 3% by weight, preferably 0.1 to 2% by weight,
(c) 5 to 95% by weight, preferably 20 to 80% by weight, even more preferably 35 to 70% by weight, in particular 45 to 55% by weight,
(d) 0 to 20% by weight, preferably 0 to 10% by weight, and
(e) 1 to 20% by weight, preferably 2 to 15% by weight, in particular 3 to 9% by weight.

9. Composite composition according to one of Claims 1 to 5 or 8, **characterized in that** the amount of releasable fluoride ions is less than 5 ppm.

10. Composite composition according to one of Claims 1 to 5, 8 or 9, **characterized in that** the acid-functional polymers are selected from polymaleic acid, polyacrylic acid, polyitaconic acid, mixtures thereof, maleic acid/acrylic acid copolymers, acrylic acid/itaconic acid copolymers and polyphosphonic acid.

## Revendications

1. Composition de composite à usage dentaire, comportant
(a) un ou plusieurs monomères polymérisables éthyléniques insaturés à base de (méth)acrylates mono-, bi- ou polyfonctionnels,
(b) des excitants de réaction habituels, et éventuellement des activateurs habituels,
(c) des matériaux de remplissage habituels,
(d) éventuellement des pigments, des adjuvants opaques aux rayons X, des agents auxiliaires thixotropiques, des émollients, et d'autres agents accessoires habituels,
**caractérisée en ce qu'**en outre
(e) elle comporte un ou plusieurs sels provenant de polymères présentant une fonction acide avec des cations à une ou plusieurs valences, éventuellement en liaison avec des verres inorganiques, c'est-à-dire sous la forme de ciments ionomères de verre durcis, les sels selon (e) se présentant sous la forme d'une poudre broyée, laquelle est mélangée avec la formulation des composites.

2. Composition de composite selon la revendication 1, **caractérisée en ce que** le composant (e) est un sel de Na, Ca, Al, Zn, Y et/ou La d'un acide carboxylique polymère, sélectionné parmi le groupe comprenant acide polymaléique, acide polyacrylique, acide polyitaconique, copolymères d'acide maléique/acide acrylique et/ou copolymères d'acide acrylique/acide itaconique, ou un acide polyphosphonique.

3. Composition de composite selon la revendication 1, **caractérisée en ce que** le composant (e) est un ciment ionomère de verre durci, de préférence à base de verres de fluorosilicate de calcium-aluminium ou de strontium-aluminium habituels.

4. Composition de composite selon l'une quelconque des revendications 1 à 3, **caractérisée en ce que** l'élément (e) a été broyé à une petite dimension de grains inférieure à 20 µm.

5. Composition de composite selon l'une quelconque des revendications 1 à 4, **caractérisée en ce que** l'élément (e) est présent dans une quantité de 1 à 20 % en poids par rapport au poids total de la composition.

6. Utilisation de la composition de composite selon l'une quelconque des revendications 1 à 5, pour la fabrication de matériaux pour des prothèses, des provisoires, et de matériaux de revêtement et de remplissage, ou pour la fabrication de matériaux synthétiques pour des appareils orthopédiques maxillaires, des dents artificielles, une structure de moignon, ou bien des ciments.

7. Utilisation de sels provenant de polymères présentant une fonction acide avec des cations à une ou plusieurs valences, en particulier de ciments ionomères de verre durcis, selon l'une quelconque des revendications 1 à 5, pour la fabrication, avec des compositions de composite à usage dentaire, de compléments inhibiteurs de plaque dentaire.

8. Compositions de composite selon l'une quelconque des revendications 1 à 5, comportant les composants (a) à (e) dans les quantités suivantes :
(a) de 4 à 68,99 % en poids, de préférence de 45 à 55 % en poids,
(b) de 0,01 à 3 % en poids, de préférence de 0,1 à 2 % en poids,
(c) de 5 à 95 % en poids, de préférence de 20 à 80 % en poids, et encore plus de préférence de 35 à 70 % en poids, en particulier de 45 à 55 % en poids,
(d) de 0 à 20 % en poids, de préférence de 0 à 10 % en poids, et
(e) de 1 à 20 % en poids, de préférence de 2 à 15 % en poids, en particulier de 3 à 9 % en poids.

9. Composition de composite selon l'une quelconque des revendications 1 à 5 ou 8, **caractérisée en ce que** la quantité d'ions fluorures libérable est inférieure à 5 ppm.

10. Composition de composite selon l'une quelconque des revendications 1 à 5, 8 ou 9, **caractérisée en ce que** les polymères présentant une fonction acide sont sélectionnés parmi le groupe comprenant acide polymaléique, acide polyacrylique, acide polyitaconique, des mélanges de ceux-ci, des copolymères d'acide maléique/acide acrylique, des copolymères d'acide acrylique/acide itaconique, et un acide polyphosphonique.
